# EUROPÄISCHE PATENTSCHRIFT

(11) **EP 2 909 621 B1**
(45) Veröffentlichungstag und Bekanntmachung des Hinweises auf die Patenterteilung: **05.07.2017**
(21) Anmeldenummer: 13780123.9
(22) Anmeldetag: 21.10.2013
(51) Int. Cl.: G01N 33/28, G01N 15/06

(54) **MESSVORRICHTUNG UND -VERFAHREN ZUR DETEKTIERUNG FERROMAGNETISCHER PARTIKEL**
DEVICE AND METHOD TO DETECT FERROMAGNETIC PARTICLES
DISPOSITIF ET MÉTHODE POUR DÉTECTER DES PARTICULES FERROMAGNÉTIQUES

(30) Priorität: 22.10.2012 DE 102012219242
(43) Veröffentlichungstag der Anmeldung: 26.08.2015
(73) Patentinhaber: Rolls-Royce Deutschland Ltd & Co KG, 15827 Blankenfelde-Mahlow (DE)
(72) Erfinder: LÜCK, Rudolf, 14558 Nuthetal (DE); HÖHNE, Peter, 15370 Fredersdorf (DE)
(86) Internationale Anmeldenummer: PCT/EP2013/071955
(87) Internationale Veröffentlichungsnummer: WO 2014/064043

(56) Entgegenhaltungen:
- DE-A1- 19 524 353
- DE-A1-102009 022 443
- FR-A1- 2 443 691
- US-A- 5 179 346
- US-A- 5 406 208
- US-A1- 2006 125 487

## Beschreibung

Die Erfindung betrifft eine Messvorrichtung zur Detektierung ferromagnetischer Partikel mit den Merkmalen des Anspruchs 1 und ein Messverfahren zur Detektierung ferromagnetischer Partikel mit den Merkmalen des Anspruchs 12.

Während des Betriebes mechanischer Vorrichtungen, wie z.B. Flugzeugtriebwerken, Getrieben oder Pumpen, fallen durch Abrieb immer wieder metallische, insbesondere ferromagnetische Partikel an, die sich in Flüssigkeiten, wie z.B. Schmiermitteln oder Hydraulikflüssigkeit ansammeln können.

Der Anfall ferromagnetischer Partikel (z.B. Eisenpartikeln) kann ein Zeichen für eine Fehlfunktion oder fortschreitenden Verschleiß in der Vorrichtung sein, die früher oder später zum Versagen der Vorrichtung führt. Der Abrieb kann z.B. zum Verstopfen von Ventilen führen.

Gerade bei Vorrichtungen, die dauerhaft einen sicheren Betrieb gewährleisten müssen - wie z.B. Flugzeugtriebwerken - ist es problematisch, dass der Abrieb der ferromagnetischen Partikel von außen nicht ohne weiteres detektierbar ist. An sich bekannte magnetische Abriebdetektoren (Magnetic Chip Detectors) weisen einen zu kleinen Messbereich für die ferromagnetischen Partikel auf.

Aus der US 5 179 346 A ist eine Messvorrichtung zur Detektierung ferromagnetischer Partikel bekannt.

Es besteht daher die Aufgabe, eine effiziente und sichere Vorrichtung zur Ermittlung des ferromagnetischen Abriebs zu entwickeln.

Die Aufgabe wird durch eine Messvorrichtung mit den Merkmalen des Anspruchs 1 gelöst.

Dabei liegen die ferromagnetischen Partikel beweglich in einem Flüssigkeitsvolumen vor, wobei ein Magnetmittel der Erzeugung eines magnetischen Feldes in dem Flüssigkeitsvolumen dient.

Ein Messmittel für den elektrischen Widerstand des Flüssigkeitsvolumens mit den darin befindlichen ferromagnetischen Partikeln erfasst den elektrischen Gesamtwiderstand, der sich insbesondere bei zunehmender Konzentration der angelagerten ferromagnetischer Partikel an die Kontakte des EMCD (Electric Magnetic Chip Detektor) ändert. Ferner weist ein Mittel zur Funktionsprüfung der Messvorrichtung mindestens einen niederohmigen Widerstand zwischen 50 und 500 Ω zur Durchführung einer Widerstandsmessung auf. Durch die "An-und Abschaltbarkeit" der Widerstände durch Umpolung oder Wechsel-Gleichspannung wird eine Funktionsprüfung gewährleistet, die die Empfindlichkeit der Messung nicht nachteilig beeinflusst und somit auch sehr kleine ferromagnetische Partikel erfassbar sind.

Die ferromagnetischen Partikel ordnen sich entlang der magnetischen Feldlinien in dem Flüssigkeitsvolumen an. Bei einer Brückenbildung der ferromagnetischen Partikel zwischen den magnetischen Polen erfolgt eine Änderung des Gesamtwiderstandes des Flüssigkeitsvolumens, der Aussagen über das Vorhandensein und die zeitliche Entwicklung des metallischen Abriebs erlaubt.

Erfindungsgemäß weist das Mittel zur Funktionsprüfung passive Schaltelemente auf und das Mittel zur Funktionsprüfung erzeugt durch das Umpolen einer Gleichspannung oder das Anlegen einer Wechselspannung an das Mittel zur Funktionsprüfung ein Signal, um eine korrekte Funktion und / oder eine Fehlfunktion der Messvorrichtung zu signalisieren.

So weist das Mittel zur Funktionsprüfung eine Steckerverbindung auf, bei der ein erstes Steckerelement mit dem Messmittel für den elektrischen Widerstand und ein zweites Steckerelement mit einer Messsonde für das Flüssigkeitsvolumen verbindbar ist, wobei das erste und zweite Steckerelement jeweils eine Reihenschaltung eines elektrischen Widerstandes mit einer Diode aufweist oder wobei das erste und zweite Steckerelement jeweils eine Reihenschaltung eines Kondensators mit einem elektrischen Widerstand aufweist. Durch das Umschalten von Gleich- auf Wechselstrom kann hierdurch eine einfache Funktionsprüfung erfolgen.

Vorteilhaft ist es, wenn die Messung des elektrischen Gesamtwiderstandes über das Flüssigkeitsvolumen in einer flüssigkeitsgefüllten Leitung mit darin angeordneten ferromagnetischen Partikel erfolgt, insbesondere in einer Schmiermittel- oder Ölleitung. Besonders vorteilhaft ist es, wenn die ferromagnetischen Partikel zur Detektion von Abrieb in einer Schmiermittel- oder Ölleitung angeordnet sind, die mit einem Wälzlager, insbesondere mit keramischen Wälzkörpern, einem Getriebe und / oder einer Pumpe gekoppelt ist.

Zusätzlich oder alternativ ist es vorteilhaft, wenn die ferromagnetischen Partikel in einer Rückführleitung für Schmiermittel, insbesondere Öl, Kühlmittel, Hydraulikflüssigkeit oder Brennstoff vor einem Filter angeordnet sind, da hier die Detektierung metallischer Partikel von besonderer Bedeutung ist.

Von Vorteil ist die Messvorrichtung so eingerichtet und ausgebildet, dass Partikel mit einem durchschnittlichen Durchmesser von weniger als 40 µm, insbesondere weniger als 20 µm, ganz insbesondere weniger als 10 µm messbar sind.

Bei dem Anfall des Abriebes ändert sich der elektrische Widerstand in weiten Grenzen, so dass es vorteilhaft ist, wenn der Messbereich des elektrischen Gesamtwiderstandes des Flüssigkeitsvolumens mit den ferromagnetischen Partikeln zwischen 1 und 10¹⁰ Ω beträgt.

Auch ist es vorteilhaft, wenn bei der Bestimmung des elektrischen Gesamtwiderstandes im Flüssigkeitsvolumen bei der Unterschreitung eines vorgegebenen Schwellenwertes ein Signal, insbesondere ein Warnsignal, über eine Fehlfunktion auslösbar ist. Damit kann z.B. eine Messung des zeitlichen Verlaufes des elektrischen Gesamtwiderstandes im Betrieb einer Vorrichtung dazu dienen, die Vorrichtung rechtzeitig abzuschalten.

Vorteilhafterweise weist das Magnetmittel zur Erzeugung des magnetischen Feldes in dem Flüssigkeitsvolumen einen Dauermagneten und / oder einen Elektromagneten auf.

Ferner ist es vorteilhaft, eine mechanische Vorrichtung, insbesondere ein Flugzeugtriebwerk, ein Getriebe, ein Wälzlager oder eine Pumpe mit einer solchen Messvorrichtung auszustatten.

Es ist auch vorteilhaft, wenn das Magnetmittel zur Erzeugung des magnetischen Feldes einen Elektromagneten aufweist, der gezielt magnetisierbar oder entmagnetisierbar ist. Damit kann z.B. im Betrieb eine Nullung des Messmittels vorgenommen werden und auch eine Reinigung ermöglicht werden.

In einer vorteilhaften Ausgestaltung wird mit dem Messmittel zur Bestimmung des elektrischen Widerstandes der zeitliche Verlauf, insbesondere der Gradient des Widerstandes über der Zeit erfasst. Durch diese Erfassung des kontinuierlichen Verlaufes des Signals über die Widerstandsänderung wird eine deutliche Verbesserung des Informationsgehaltes erreicht, da die Zuwachsrate (Verringerung des Widerstandes über die Zeit), als auch die Stufung des Zuwachses zu erkennen ist (Treppenhöhe).

Die Aufgabe wird auch durch ein Verfahren zur Detektierung ferromagnetischer Partikel in einem Flüssigkeitsvolumen gelöst, wobei mit dem Messmittel für den elektrischen Gesamtwiderstand des Flüssigkeitsvolumens mit den darin befindlichen metallischen Partikeln der zeitliche Verlauf des elektrischen Gesamtwiderstandes in einer Vorrichtung erfasst wird. Dabei wird vorteilhafterweise bei der Überwachung des zeitlichen Verlaufes in Abhängigkeit von einem vorgegebenen Schwellenwert ein Signal, insbesondere ein Warnsignal, über eine Fehlfunktion ausgelöst.

In einer vorteilhaften Ausgestaltung weist das Magnetmittel zur Erzeugung des magnetischen Feldes einen Elektromagneten mit einem ferromagnetischen Kern auf, wobei zu Zwecken der Reinigung der Strom abgeschaltet wird und mit einem Wechselstrom mit kleiner werdender Amplitude eine Entmagnetisierung des Kernes erfolgt. Auch ist es damit möglich, dass vor dem Abschalten des elektrischen Stromes der ferromagnetische Kern noch magnetisiert werden kann, um die Entnahme von ferromagnetischen Partikeln nach dem Abschalten des Stromes zu gewährleisten.

In Zusammenhang mit den in den Figuren dargestellten Ausführungsbeispielen wird die Erfindung erläutert. Dabei zeigt
- Fig. 1: eine schematische Darstellung einer Messung in einer Flüssigkeitsleitung mit ferromagnetischen Partikeln über einem Magneten, die elektrischen Kontakte verbindend:
- Fig. 1A: eine Vergrößerung eines Ausschnittes aus der Anlagerung von ferromagnetischen Partikeln aneinander im magnetischen Feld in einem Flüssigkeitsvolumen gemäß Fig. 1;
- Fig. 2: eine schematische Darstellung einer ersten Ausführungsform der Beschaltung für eine Steckverbindung;
- Fig. 3: eine schematische Darstellung einer zweiten Ausführungsform der Beschaltung für eine Steckverbindung;
- Fig. 4: Darstellung einer Messung des Gesamtwiderstandes in einem Flüssigkeitsvolumen;
- Fig. 5: eine Ausführungsform der Messvorrichtung mit einem Elektromagneten.

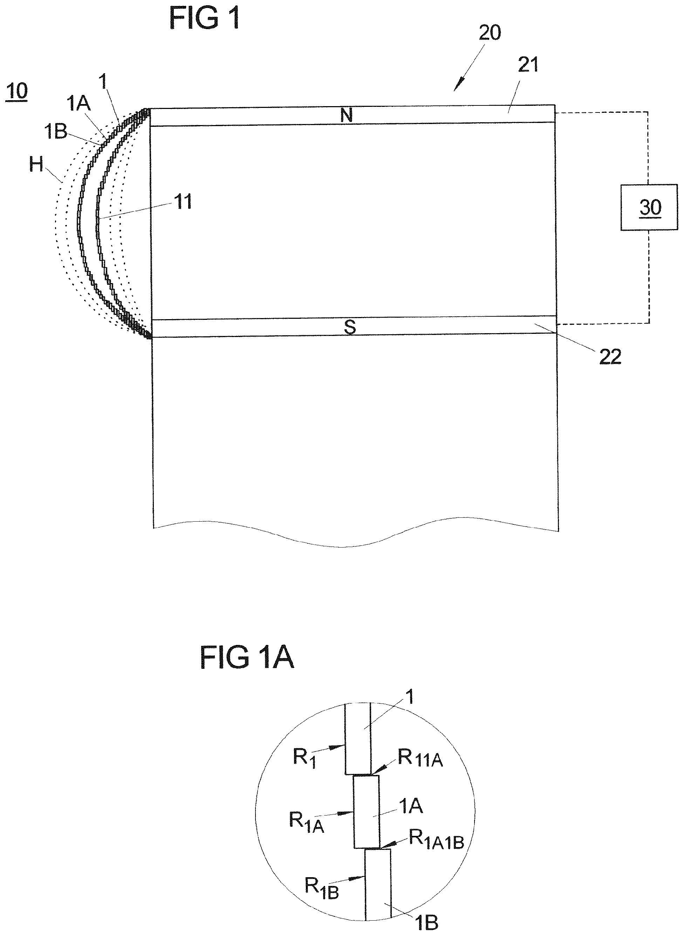

In Fig. 1 ist eine mit Schmieröl gefüllte Leitung 10 als Flüssigkeitsvolumen dargestellt, in der sich ferromagnetische Partikel 1, 1A, 1B (z.B. Eisen oder Stahlpartikel) befinden (siehe Vergrößerung in Fig. 1A). Alternativ können sich solche ferromagnetischen Partikel 1, 1A, 1B auch in Hydraulikleitungen ansammeln.

Solche ferromagnetischen Partikel 1, 1A, 1B können sich infolge normalen Abriebs oder auch durch Schädigungen in einer hier nicht dargestellten Vorrichtung bilden. So kann z.B. in einem hybriden Wälzlager (Metall-Laufbahnen mit keramischen Kugeln oder Rollen) der Ausbruch an einem keramischen Wälzkörpers zu einer Schädigung der metallenen Laufbahn des Wälzkörpers führen. So gelangen beispielsweise ferromagnetische Partikel 1, 1A, 1B in den Schmiermittelkreislauf und damit in das Flüssigkeitsvolumen einer Leitung 10.

Wird mit einem Magnetmittel 20 ein magnetisches Feld H an die Flüssigkeit in der Leitung 10 angelegt, so ordnen sich die ferromagnetischen Partikel 1, 1A, 1B entlang der Feldlinien zwischen den Magnetpolen 21, 22 in dem Flüssigkeitsvolumen der Leitung 10 an. Grundsätzlich kann der Magnetmittel 20 als ein Stabmagnet aufgefasst werden, der in die Flüssigkeit 10 hineinragt. Konstruktiv kann dies dadurch gelöst werden, dass ein solcher Stabmagnet mittels einer Hülse z.B. in einen Ölkanal oder eine Hydraulikleitung eingeschraubt wird. An dem der Flüssigkeit 10 abgewandeten Seite sind Leitungen angeordnet, die Messsignale aus der Flüssigkeit 10 übertragen, z.B. an ein Messmittel 30 zur Bestimmung des elektrischen Widerstandes.

Ab einer gewissen Anzahl der ferromagnetischen Partikel 1, 1A, 1B ordnen sich die ferromagnetischen Partikel 1, 1A, 1B entlang der magnetischen Feldlinien H in der Flüssigkeit 10 in Ketten an, so dass elektrisch leitende Brücken 11 entstehen, wie dies in Fig. 1 schematisch dargestellt ist. Wird nun der elektrische Gesamtwiderstand oder die elektrische Leitfähigkeit des Inhalts der Leitung 10 mit dem Messmittel 30 gemessen, lässt sich die Ausbildung der Brücken 11 detektieren, da der elektrische Widerstand sinkt (oder die Leitfähigkeit steigt).

Der elektrische Gesamtwiderstand der Brücke 11 setzt sich aus dem ohmschen Widerstand R₁, R_{1A}, R_{1B} der einzelnen ferromagnetischen Partikel 1, 1A, 1B und den Übergangswiderständen R₁, R_{1A}, R_{1A1B} zwischen den ferromagnetischen Partikeln 1, 1A, 1B etc. zusammen. Bei noch nicht vollständig mit ferromagnetischen Partikeln überbrückten Kontakten, fließt der Strom über die Flüssigkeit, allerdings mit einem sehr viel höheren Widerstand.

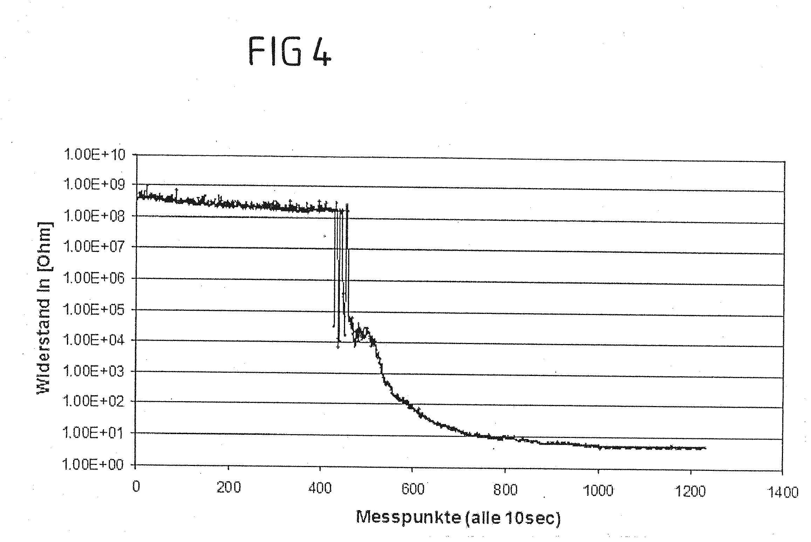

Mit der hier beschriebenen Ausführungsform ist es möglich, die Änderung des elektrischen Widerstands (siehe Fig. 4) durch die Bildung sehr kleiner ferromagnetischer Partikel 1, 1A, 1B (insbesondere kleiner 50 µm) zu messen. Die ferromagnetischen Partikel 1, 1A, 1B formieren sich dabei entlang des magnetischen Felds H. Bei den hier dargestellten Ausführungsformen können die ferromagnetischen Partikel 1, 1A, 1B nahezu beliebig klein sein. Die Grenze ist dort, wo die magnetisch wirksame Kraft kleiner wird als die Widerstandskraft. Die Widerstandskraft ist gegeben durch die Oberfläche des ferromagnetischen Partikels 1, 1A, 1 B, die Viskosität der Flüssigkeit (z.B. eines Öles) und die Geschwindigkeit des ferromagnetischen Partikels 1, 1A, 1B, hervorgerufen durch die magnetische Kraft in Richtung des Magneten. Die ferromagnetischen Partikel 1, 1A, 1B müssen den Magneten in der Zeit erreichen (den Partikel durch die Flüssigkeit anziehen), in der der Partikel an dem Magneten vorbeizieht. Die Relation magnetisch wirksamer Masse zu effektiver Oberfläche des Partikels ist von Bedeutung, zusammen mit der Geschwindigkeit des Ölflusses und der Viskosität des Öles.

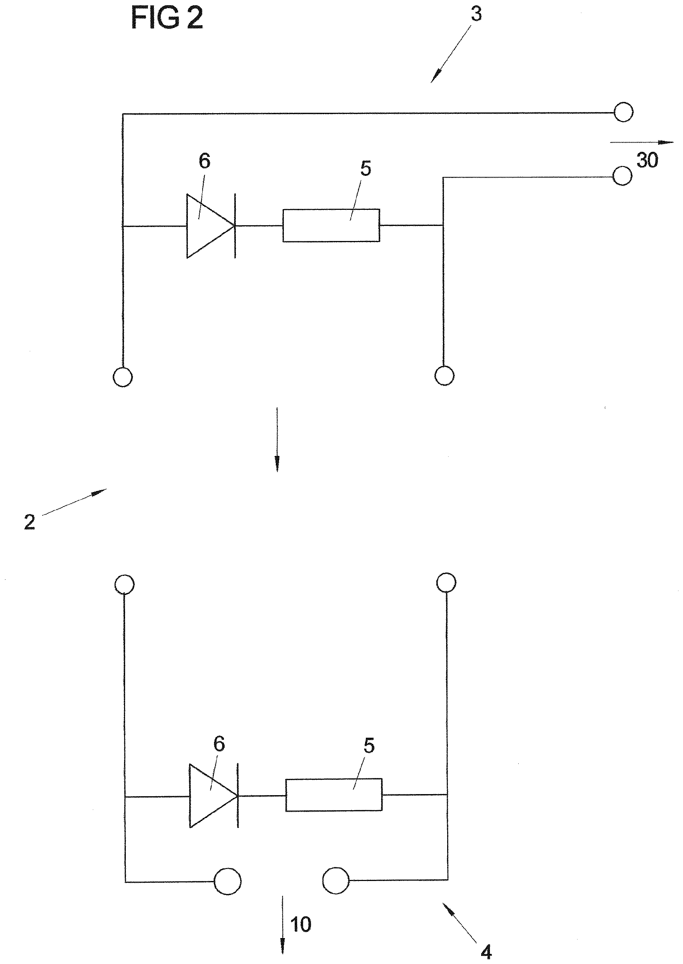

In Fig. 2 ist in schematischer Weise eine erste Ausführungsform der Beschaltung einer Steckerverbindung 3, dargestellt, die als Mittel 2 zur Funktionsprüfung der Messvorrichtung zur Detektierung der ferromagnetischen Partikel 1, 1A, 1B ausgebildet ist. Das Steckerelement 3 ist dabei dem Messmittel 30 zur Bestimmung des elektrischen Widerstandes zugeordnet. Über das zweite Steckerelement 4 erfolgt eine Messung des elektrischen Widerstandes im Flüssigkeitsvolumen 10.

Dabei sind die beiden Steckerelemente 3, 4 des Mittels 2 zur Funktionsprüfung mit passiven elektronischen Bauelementen, hier jeweils mit einem elektrischen Widerstand 5 und einer Diode 6, versehen. Eine Funktionsprüfung dieser ersten Ausführungsform erfolgt durch eine Umpolung einer Gleichspannung. Die Diode 6 arbeitet nur in einer Richtung in Durchlassrichtung, so dass einmal ein sehr hoher Widerstand (Sperrwiderstand) und einmal nur der Durchlasswiderstand der Diode zusätzlich zu dem ohmschen Widerstand in den Steckern 3, 4 vorliegt.

Im Messbetrieb wird die Diode 6 in Sperrrichtung betrieben, so dass der Widerstand in den Steckerelementen 3, 4 keine Rolle spielt. Die Steckerverbindung erlaubt somit eine genaue Messung über einen sehr großen Widerstandsbereich, wie dies anhand der beispielhaften Messkurve in Fig. 4 belegt wird. Bei der Messung bis zum Messpunkt 400 fließt der Strom über die Flüssigkeit. Die elektrische Leitfähigkeit ändert sich erkennbar mit dem Zusammenwachsen der Partikelanhaftungen am Nord- und Südpol 21, 22 des Magnetmittels 20. In diesem Fall wird der elektrische Strom zum Teil in der Flüssigkeit fließen (siehe Fig. 4, bis zum Messpunkt 400). Wenn die Partikelbelage am Nord- und Südpol 21, 22 des Magnetmittels 20 sich berühren, geht der Widerstand zwischen den elektrischen Kontakten um mehrere Dekaden herunter.

Ab dem Messpunkt 420 berühren sich die angelagerten Metallpartikel von Nord- und Südpol und der Strom fließ über die ferromagnetischen Partikel und den Übergangswiderstand zwischen den Partikeln 1, 1A, 1B.

Der im Flüssigkeitsvolumen 10 gemessene elektrische Widerstand ergibt sich aus der Summe der einzelnen elektrischen Widerstände R_{A}, R_{AA1}, R_{A1}, R_{1A1B}, R_{1B} der ferromagnetischen Partikel 1, 1A, 1B, etc. wobei sich die ferromagnetischen Partikel 1, 1A, 1B in Art einer leitfähigen Brücke 11 zwischen den magnetischen Polen 21, 22 anordnen, und dem Widerstand über die Flüssigkeit (Messpunkte 0 bis 400), bevor die Brücke über die Kontakte vollständig ausgebildet ist.

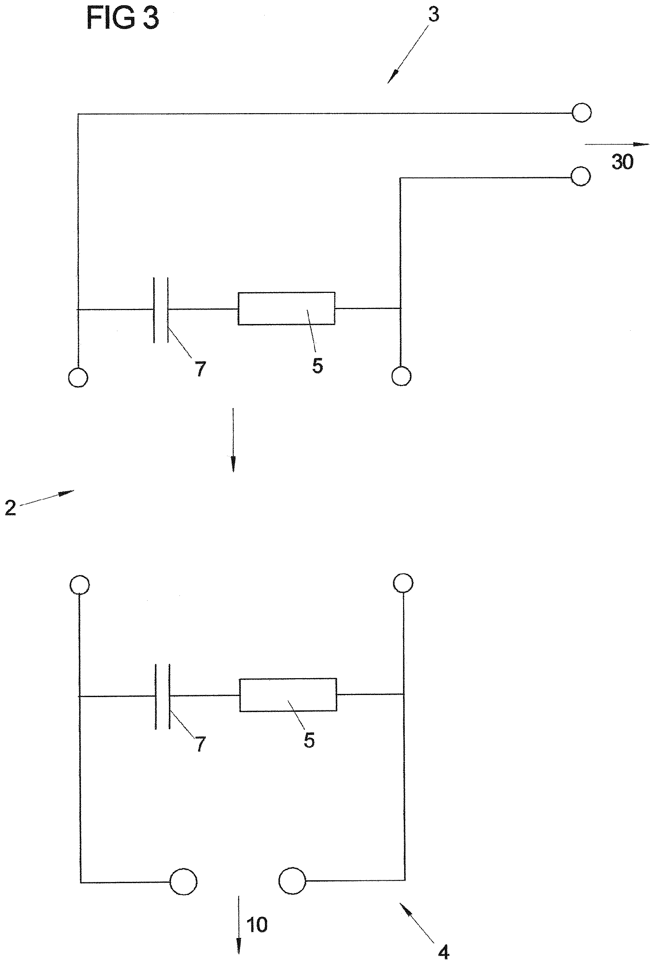

Die zweite Ausführungsform gemäß der Fig. 3 funktioniert in ähnlicher Weise. Allerdings sind die Steckerelemente 3, 4 hier jeweils mit in Reihe geschalteten Widerständen 5 und Kondensatoren 7 ausgerüstet. In zusammengesetztem Zustand liegt in der Steckverbindung eine Reihenschaltung von jeweils einem Widerstand 5 und einem Kondensator 7 vor.

Die Funktionsprüfung erfolgt dabei, indem auf eine Wechselspannung umgeschaltet wird. Dabei wird der komplexe Widerstand, bestehend aus den Widerständen 5 und den Kondensatoren 7, sichtbar.

Im Messbetrieb unter Gleichspannung gibt der gemessene Widerstand den Gesamtwiderstand der ferromagnetischen Partikel 1, 1A, 1B in dem Flüssigkeitsvolumen 10 wieder. Auch hiermit lassen sich sehr genaue Messungen über einen großen Widerstandsbereich durchführen.

Mit den beschriebenen Ausführungsformen des Mittels 2 zur Funktionsprüfung lassen sich z.B. folgende Fehlerfälle untersuchen:

### 1. Fehlerhafte Steckerverbindung 3, 4

Bei einem nicht oder nicht vollständig eingesteckten Steckerelement 3, 4 ergibt eine Widerstandsmessung den einfachen Widerstandswert. Bei eingesteckten Steckerelementen ergibt sich ein halbierter Widerstand (gleiche Widerstände vorausgesetzt).

### 2. Kabelbruch in der Messleitung

Der bei einem Kabelbruch gemessene elektrische Widerstand ist viel größer als der Testwiderstand aus der Parallelschaltung der Widerstände.

### 3. Masseschluss der Messleitung

Der gemessene Widerstand ist viel kleiner als der Testwiderstand aus der Parallelschaltung der Widerstände.

Die hier beschriebenen Ausführungsformen eignen sich auch für die Fälle, bei denen nicht davon ausgegangen werden kann, dass nur wenige Partikel in Serie die Kontakte über den Magnetpolen 21, 22 schließen. Dies ist vor allem dann wichtig, wenn mit Feinstabrieb wie bei Lagern, Getrieben Pumpen usw. zu rechnen ist.

Mit an sich bekannten EMCDs (Electric Magnetic Chip Detectors) konnte erst relativ spät ein hinreichender elektrischer Widerstand ermittelt werden. Hingegen können die oben beschriebenen Ausführungsformen bereits früh eine zuverlässige Indikation auf ferromagnetische Partikel 1, 1A, 1B liefern. Dies liegt unter anderem an der bisherigen Schutzbeschaltung der bekannten EMCDs. Diese Schutzbeschaltung beinhaltet unter anderem einen niedrigen Widerstand im EMCD selbst (parallel zu den Kontakten) als auch einen niedrigen Widerstand an den Steckerelementen (parallel zu den Kontakten) zum EMCD. Der niedrige Gesamtwiderstand ist dann parallel zum Summenwiderstand der ferromagnetischen Partikel 1, 1A, 1B an den Kontakten über den Magnetpolen 21, 22 des EMCDs. Dies führt in der Parallelschaltung dazu, dass hohe Summenwiderstände über den Kontakten nicht erkannt werden können. Aus diesem Grund wurden bei den bisherigen bekannten Vorrichtungen auch keine Widerstandsmessungen vorgenommen.

Mit den hier dargestellten Ausführungsformen wird auch der Betrieb eines Verfahrens zur Detektion ferromagnetischer Partikel 1, 1A, 1B ermöglicht, bei dem der Widerstandsabfall gemessen wird. Zu erwarten ist, dass mit fortschreitender Aufnahme elektrisch leitender ferromagnetischer Partikel 1, 1A, 1B der Widerstand signifikant sinkt. Wiederholte Messungen - beispielhaft an Fig. 4 dargestellt - zeigen, dass der Widerstand bei einem Kugelausbruch innerhalb von ca. 13 Minuten von 200 MΩ auf 25 kΩ sinkt. In der Folge erfolgt ein stetiger Abfall und eine asymptotische Annäherung an einen Widerstand von 4 Ω.

Unterschreitet der Widerstand einen bestimmten Wert, so kann in einer Ausführungsform automatisch ein Warnsignal generiert werden, da mit erheblichem Abriebmaterial im Flüssigkeitsvolumen 10 zu rechnen ist.

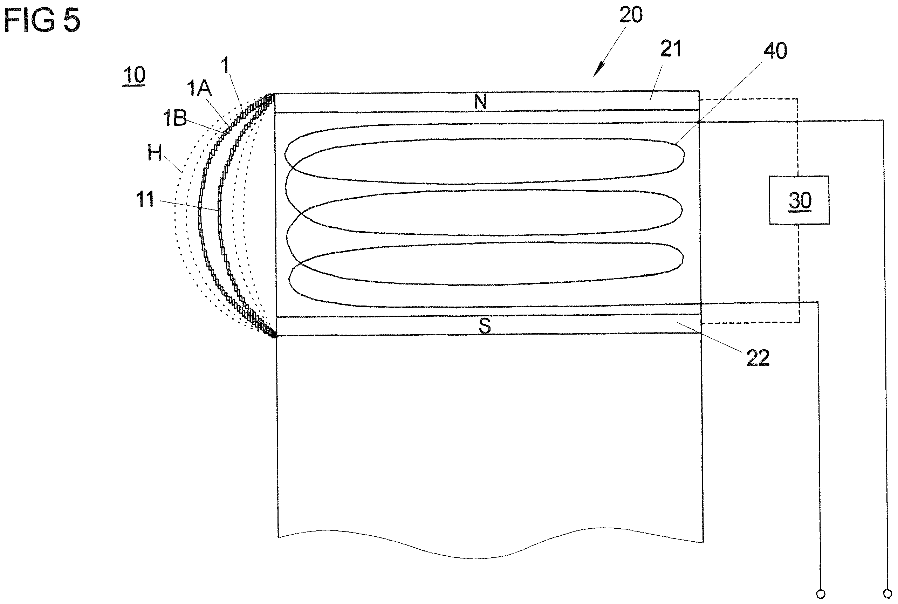

In Fig. 5 ist eine weitere Ausführungsform dargestellt. Dabei wird in der Messvorrichtung ein Magnetmittel 20 mit einem Elektromagneten eingesetzt. Dies hat den Vorteil, dass der Elektromagnet bei vollem Besatz mit ferromagnetischen Partikeln 1, 1A, 1B (d.h. bei einem kleinen elektrischen Widerstand), abgeschaltet werden kann. Zudem kann man bei dem Elektromagneten den Restmagnetismus aus dem ferromagnetischen Kern entmagnetisieren. Dies geschieht durch das Anlegen von Wechselspannung mit immer kleiner werdender Amplitude.

Als Kernmaterial 40 für den Elektromagneten bietet sich magnetisch mittelhartes Material an. Dies vereint die Vorteile, dass der Magnetismus gehalten werden kann und dass eine Entmagnetisierung problemlos möglich ist.

Damit ist es möglich, bei einem mit ferromagnetischen Partikeln voll besetzten EMCD ein Abschalten und eine Entmagnetisierung vorzunehmen. Damit kann nach dem vollen Belag wieder ein "Nullen" der Messvorrichtung herbeigeführt werden und es entfällt die Notwendigkeit des manuellen Reinigens bei der Anzeige des vollen Belages. Die Messung kann somit beliebig lange auch während des Fluges erfolgen.

Soll z.B. nach dem Abschalten eines Triebwerkes eine Entnahme der ferromagnetischen Partikel vom EMCD erfolgen, kann der EMCD, vor dem Abschalten des Stromes, mit einem höheren Strom beaufschlagt werden, was zu einer permanenten Magnetisierung des mittelharten Kernmaterials führen würde und somit die Partikel bis zur manuellen Entnahme halten würde.

### Bezugszeichenliste

- 1: ferromagnetische Partikel
- 2: Mittel zur Funktionsprüfung
- 3: erstes Steckerelement
- 4: zweites Steckerelement
- 5: Widerstand
- 6: Diode
- 7: Kondensator

- 10: Flüssigkeitsvolumen, Leitung mit Flüssigkeit
- 11: leitfähige Brücke aus ferromagnetischen Partikeln

- 20: Magnetmittel zur Erzeugung eines magnetischen Feldes
- 21: magnetischer Nordpol
- 22: magnetischer Südpol

- 30: Messmittel zur Bestimmung des elektrischen Widerstandes

- 40: Kern eines Elektromagneten

- H: magnetisches Feld

## Patentansprüche

1. Messvorrichtung zur Detektierung ferromagnetischer Partikel, die beweglich in einem Flüssigkeitsvolumen angeordnet sind, aufweisend
ein Magnetmittel (20) zur Erzeugung eines magnetischen Feldes (H) in dem Flüssigkeitsvolumen (10),
ein Messmittel (30) für den elektrischen Gesamtwiderstand des Flüssigkeitsvolumens (10) mit den darin befindlichen ferromagnetischen Partikeln (1, 1A, 1B) und
ein Mittel (2) zur Funktionsprüfung der Messvorrichtung mit mindestens einem niederohmigen Widerstand (5) zwischen 50 und 500 Ω zur Durchführung einer Widerstandsmessung,
**gekennzeichnet dadurch, dass**
- das Mittel (2) zur Funktionsprüfung eine Steckerverbindung aufweist, deren erstes Steckerelement (3) mit dem Messmittel (30) für den elektrischen Widerstand und deren zweites Steckerelement (4) mit einer Messsonde für das Flüssigkeitsvolumen (10) verbindbar ist, wobei
- das erste und zweite Steckerelement (3, 4) jeweils eine Reihenschaltung des mindestens einen elektrischen Widerstandes (5) mit einer Diode (6) aufweist, oder
- der erste und zweite Steckerelement (3, 4) jeweils eine Reihenschaltung eines Kondensators (7) mit dem mindestens einen elektrischen Widerstand (5) aufweist,
- und dass das Mittel (2) zur Funktionsprüfung durch das Umpolen einer Gleichspannung oder das Anlegen einer Wechselspannung an das Mittel (2) zur Funktionsprüfung ein Signal erzeugt, um eine korrekte Funktion oder eine Fehlfunktion der Messvorrichtung zu signalisieren.

2. Messvorrichtung nach Anspruch 1, **dadurch gekennzeichnet, dass** die Messung des elektrischen Widerstandes über das Flüssigkeitsvolumen (10) in einer flüssigkeitsgefüllten Leitung mit darin angeordneten ferromagnetischen Partikeln (1) erfolgt, insbesondere in einer Schmiermittel- oder Ölleitung.

3. Messvorrichtung nach Anspruch 2, **dadurch gekennzeichnet, dass** die ferromagnetischen Partikel (1) zur Detektion von Abrieb in einer Schmiermittel- oder Ölleitung (10) angeordnet sind, die mit einem Wälzlager, insbesondere mit keramischen Wälzkörpern, einem Getriebe, und / oder einer Pumpe gekoppelt ist.

4. Messvorrichtung nach Anspruch 2 oder 3, **dadurch h gekennzeichnet,** dass die ferromagnetischen Partikel (1) in einer Rückführleitung (10) für Schmiermittel, insbesondere Öl, Hydraulikflüssigkeit, Brennstoff und / oder Kühlflüssigkeit vor einem Filter angeordnet sind.

5. Messvorrichtung nach mindestens einem der vorhergehenden Ansprüche, **dadurch gekennzeichnet, dass** sie so eingerichtet und ausgebildet ist, Partikel mit einem durchschnittlichen Durchmesser von kleiner 40 µm, insbesondere kleiner 20 µm, ganz insbesondere kleiner als 10 µm zu messen.

6. Messvorrichtung nach mindestens einem der vorhergehenden Ansprüche, **dadurch gekennzeichnet, dass** der Messbereich des elektrischen Gesamtwiderstandes des Flüssigkeitsvolumens (10) zwischen 1 und 10¹⁰Ω beträgt.

7. Messvorrichtung nach mindestens einem der vorhergehenden Ansprüche, **dadurch gekennzeichnet, dass** bei der Bestimmung des elektrischen Gesamtwiderstandes des Flüssigkeitsvolumens (10) bei der Unterschreitung eines vorgegebenen Schwellenwertes ein Signal, insbesondere ein Warnsignal, über eine Fehlfunktion auslösbar ist.

8. Messvorrichtung nach mindestens einem der vorhergehenden Ansprüche, **dadurch gekennzeichnet, dass** das Magnetmittel (20) zur Erzeugung des magnetischen Feldes (H) in dem Flüssigkeitsvolumen (10) einen Dauermagneten und / oder einen Elektromagneten aufweist.

9. Messvorrichtung nach mindestens einem der vorhergehenden Ansprüche, **dadurch gekennzeichnet, dass** das Magnetmittel (20) zur Erzeugung des magnetischen Feldes (H) einen Elektromagneten aufweist, der gezielt magnetisierbar oder entmagnetisierbar ist.

10. Messvorrichtung nach mindestens einem der vorhergehenden Ansprüche, **dadurch gekennzeichnet, dass** mit dem Messmittel (30) zur Bestimmung des elektrischen Widerstandes der zeitliche Verlauf, insbesondere der Gradient des Widerstandes über der Zeit erfassbar ist.

11. Mechanische Vorrichtung, insbesondere ein Flugzeugtriebwerk, ein Getriebe, ein Wälzlager oder eine Pumpe mit einer Messvorrichtung nach mindestens einem der Ansprüche 1 bis 10.

12. Verfahren zur Detektierung ferromagnetischer Partikel in einem Flüssigkeitsvolumen mit einer Messvorrichtung nach mindestens einem der Ansprüche 1 bis 10,
**dadurch gekennzeichnet,**
**dass** mit dem Messmittel (30) für den elektrischen Gesamtwiderstand des Flüssigkeitsvolumens (10) mit den darin befindlichen ferromagnetischen Partikeln (1, 1A, 1 B) der zeitliche Verlauf des elektrischen Widerstandes in einer Vorrichtung erfasst wird.

13. Verfahren nach Anspruch 12, **dadurch gekennzeichnet, dass** bei der Überwachung des zeitlichen Verlaufes in Abhängigkeit von einem vorgegebenen Schwellenwert ein Signal, insbesondere ein Warnsignal, über eine Fehlfunktion ausgelöst wird.

14. Verfahren nach Anspruch 12 oder 13, **dadurch gekennzeichnet, dass** das Magnetmittel (20) zur Erzeugung des magnetischen Feldes (H) einen Elektromagneten mit einem ferromagnetischen Kern (40) aufweist, wobei zu Zwecken der Reinigung der Strom abgeschaltet wird und mit einem Wechselstrom mit kleiner werdender Amplitude eine Entmagnetisierung des Kernes erfolgt.

15. Verfahren nach Anspruch 14, **dadurch gekennzeichnet, dass** bei Verwendung eines Elektromagneten mit einem ferromagnetischen Kern (40), vor dem Abschalten des elektrischen Stromes der ferromagnetische Kern (40) noch magnetisiert werden kann, um die Entnahme von ferromagnetischen Partikeln (1, 1A, 1B) nach dem Abschalten des Stromes zu gewährleisten.

## Claims

1. Measuring device for detecting ferromagnetic particles which are movably arranged in a liquid volume, including
a magnetic means (20) for generating a magnetic field (H) in the liquid volume (10),
a measuring means (30) for the total electric resistance of the liquid volume (10) with the ferromagnetic particles (1, 1A, 1B) contained therein, and
a means (2) for testing the function of the measuring device with at least one low-impedance resistor (5) of between 50 and 500 Ω for carrying out a resistance measurement,
**characterized in that**
- the means (2) for functional testing includes a plug connection, the first connector element (3) of which being connectable with the measuring means (30) for the electric resistance, and the second connector element (4) of which being connectable with a measuring probe for the liquid volume (10), where
- the first and second connector elements (3, 4) each have a series connection of the at least one electric resistor (5) with a diode (6), or
- the first and second connector elements (3, 4) each have a series connection of a capacitor (7) with the at least one electric resistor (5),
- and that the means (2) for functional testing generates a signal by reversing the polarity of a DC voltage or by applying an AC voltage to the means (2) for functional testing, in order to indicate a correct function or a malfunction of the measuring device.

2. Measuring device in accordance with Claim 1, **characterized in that** the measurement of the electric resistance as a function of the liquid volume (10) is carried out in a liquid-filled line with ferromagnetic particles (1) arranged therein, in particular in a lubricant or oil line.

3. Measuring device in accordance with Claim 2, **characterized in that** the ferromagnetic particles (1) for detecting wear are arranged in a lubricant or oil line (10), which is coupled to a rolling bearing, in particular with ceramic rolling elements, to a gear and/ or a pump.

4. Measuring device in accordance with Claim 2 or 3, **characterized in that** the ferromagnetic particles (1) are arranged in a return line (10) for lubricants, in particular oil, hydraulic fluid, fuel and/ or cooling liquid upstream of a filter.

5. Measuring device in accordance with at least one of the preceding Claims, **characterized in that** the device is configured and designed such that particles having an average diameter of less than 40 µm, particularly less than 20 µm, more particularly less than 10 µm can be measured.

6. Measuring device in accordance with at least one of the preceding Claims, **characterized in that** the measuring range of the total electric resistance of the liquid volume (10) is between 1 and 10¹⁰ Ω.

7. Measuring device in accordance with at least one of the preceding Claims, **characterized in that** during determination of the total electric resistance of the liquid volume (10) a signal, in particular a warning signal can be triggered when a predetermined threshold value is undershot, in order to indicate a malfunction.

8. Measuring device in accordance with at least one of the preceding Claims, **characterized in that** the magnetic means (20) for generating the magnetic field (H) in the liquid volume (10) has a permanent magnet and/ or a solenoid.

9. Measuring device in accordance with at least one of the preceding Claims, **characterized in that** the magnetic means (20) for generating the magnetic field (H) has a solenoid that can be selectively magnetized or demagnetized.

10. Measuring device in accordance with at least one of the preceding Claims, **characterized in that** using the measuring means (30) for the determination of the electric resistance it is possible to determine the time profile, in particular the gradient of the resistance as a function of time.

11. Mechanical device, in particular an aircraft engine, a gear, a rolling bearing or a pump having a measuring device in accordance with at least one of the Claims 1 to 10.

12. Method for detecting ferromagnetic particles in a liquid volume by means of a measuring device in accordance with at least one of the Claims 1 to 10,
**characterized in that**
using the measuring means (30) for the total electric resistance of the liquid volume (10) with the ferromagnetic particles (1, 1 A, 1B) contained therein, the time profile of the electric resistance in a device is determined.

13. Method in accordance with Claim 12, **characterized in that** when monitoring the time profile, a signal, in particular a warning signal is triggered as a function of a predetermined threshold value, to indicate a malfunction.

14. Method in accordance with Claim 12 or 13, **characterized in that** the magnetic means (20) for generating the magnetic field (H) includes a solenoid with a ferromagnetic core (40), where the current is switched off for cleaning purposes and the core is demagnetized using an alternating current with decreasing amplitude.

15. Method in accordance with Claim 14, **characterized in that** when using a solenoid with a ferromagnetic core (40), the ferromagnetic core (40) can still be magnetized before the electric current is switched off, in order to ensure the removal of ferromagnetic particles (1, 1A, 1B) after switching off the current.

## Revendications

1. Dispositif de mesure pour détecter des particules ferromagnétiques qui sont en mouvement dans un volume de liquide, présentant
un moyen magnétique (20) servant à générer un champ magnétique (H) dans le volume de liquide (10),
un moyen de mesure (30) pour la résistance totale électrique du volume de liquide (10) avec les particules ferromagnétiques (1, 1A, 1B) se trouvant dedans et
un moyen (2) pour tester le fonctionnement du dispositif de mesure avec au moins une résistance de basse impédance (5) comprise entre 50 et 500 Ω pour réaliser une mesure de résistance,
**caractérisé en ce que**
- le moyen (2) pour tester le fonctionnement présente une connexion enfichable dont le premier élément connecteur (3) peut être relié au moyen de mesure (30) pour la résistance électrique et dont le second élément connecteur (4) peut être relié à une sonde de mesure pour le volume de liquide (10), sachant que
- le premier et le second élément connecteur (3, 4) présentent chacun un branchement en série de l'au moins une résistance électrique (5) avec une diode (6), ou que
- le premier et le second élément connecteur (3, 4) présentent chacun un branchement en série d'un condensateur (7) avec l'au moins une résistance électrique (5),
- et que le moyen (2) pour tester le fonctionnement génère, en inversant la polarité d'une tension continue ou en appliquant une tension alternative au moyen (2) pour tester le fonctionnement, un signal afin d'indiquer un fonctionnement correct ou un dysfonctionnement du dispositif de mesure.

2. Dispositif de mesure selon la revendication n° 1, **caractérisé en ce que** la mesure de la résistance électrique en fonction du volume de liquide (10) est réalisée dans une conduite remplie de liquide avec des particules ferromagnétiques (1) disposées dedans, notamment dans une conduite de lubrifiant ou d'huile.

3. Dispositif de mesure selon la revendication n° 2, **caractérisé en ce que** les particules ferromagnétiques (1) servant à la détection de l'abrasion sont disposées dans une conduite de lubrifiant ou d'huile (10) qui est accouplée à un palier à roulement, notamment à des rouleaux en céramique, à un engrenage et/ ou à une pompe.

4. Dispositif de mesure selon la revendication n° 2 ou n° 3, **caractérisé en ce que** les particules ferromagnétiques (1) sont disposées dans une conduite de retour (10) pour lubrifiant, notamment pour huile, liquide hydraulique, carburant et/ ou liquide de refroidissement, en amont d'un filtre.

5. Dispositif de mesure selon au moins une des revendications précédentes, **caractérisé en ce que** celui-ci est configuré et conçu de manière à mesurer des particules avec un diamètre moyen de moins de 40 µm, notamment moins de 20 µm, et tout particulièrement moins de 10 µm.

6. Dispositif de mesure selon au moins une des revendications précédentes, **caractérisé en ce que** la plage de mesure de la résistance électrique totale du volume de liquide (10) est comprise entre 1 et 10¹⁰ Ω.

7. Dispositif de mesure selon au moins une des revendications précédentes, **caractérisé en ce que**, lors de la détermination de la résistance électrique totale du volume de liquide (10), un signal, notamment un signal d'avertissement, peut être déclenché en cas de dépassement d'une valeur limite prédéfinie vers le bas pour signaler un dysfonctionnement.

8. Dispositif de mesure selon au moins une des revendications précédentes, **caractérisé en ce que** le moyen magnétique (20) servant à générer le champ magnétique (H) dans le volume de liquide (10) présente un aimant permanent et/ ou un électroaimant.

9. Dispositif de mesure selon au moins une des revendications précédentes, **caractérisé en ce que** le moyen magnétique (20) servant à générer le champ magnétique (H) présente un électroaimant qui peut être magnétisé ou démagnétisé de manière sélective.

10. Dispositif de mesure selon au moins une des revendications précédentes, **caractérisé en ce qu'**il est possible avec le moyen de mesure (30) pour déterminer la résistance électrique d'acquérir la progression dans le temps, notamment le gradient de la résistance en fonction du temps.

11. Dispositif mécanique, notamment un moteur d'avion, un engrenage, un palier à roulement ou une pompe avec un dispositif de mesure selon au moins une des revendications n° 1 à n° 10.

12. Procédé pour la détection de particules ferromagnétiques dans un volume de liquide avec un dispositif de mesure selon au moins une des revendications n° 1 à n° 10,
**caractérisé en ce que**
la progression dans le temps de la résistance électrique dans un dispositif est acquise en utilisant le moyen de mesure (30) pour la résistance électrique totale du volume de liquide (10) avec les particules ferromagnétiques (1, 1A, 1 B) se trouvant dedans.

13. Procédé selon la revendication n° 12, **caractérisé en ce que** lors de la surveillance de la progression dans le temps, un signal, notamment un signal d'avertissement, est déclenché en fonction d'une valeur limite prédéfinie, afin d'indiquer un dysfonctionnement.

14. Procédé selon la revendication n° 12 ou n° 13, **caractérisé en ce que** le moyen magnétique (20) pour générer le champ magnétique (H) présente un électroaimant avec un noyau ferromagnétique (40), sachant que le courant est coupé à des fins de nettoyage et qu'une démagnétisation du noyau a lieu au moyen d'un courant alternatif d'amplitude décroissante.

15. Procédé selon la revendication n° 14, **caractérisé en ce que**, lors de l'utilisation d'un électroaimant avec un noyau ferromagnétique (40), le noyau ferromagnétique (40) peut encore être magnétisé avant la coupure du courant électrique pour permettre le prélèvement de particules ferromagnétiques (1, 1A, 1 B) après la coupure du courant.
